# EUROPEAN PATENT SPECIFICATION

(11) **EP 1 482 936 B1**
(45) Date of publication and mention of the grant of the patent: **04.10.2006**
(21) Application number: 03711914.6
(22) Date of filing: 03.03.2003
(51) Int. Cl.: A61K 31/436, A61K 47/14, A61K 47/44, A61K 31/4353

(54) **OPHTHALMIC COMPOSITION COMPRISING ASCOMYCIN**
OPHTHALMISCHE ZUBEREITUNG MIT ASCOMYCIN
COMPOSITION OPHTALMIQUE A BASE D'ASCOMYCINE

(30) Priority: 04.03.2002 US 361515 P; 09.09.2002 US 409275 P
(43) Date of publication of application: 08.12.2004
(73) Proprietor: Novartis AG, 4056 Basel (CH); Novartis Pharma GmbH, 1235 Vienna (AT)
(72) Inventor: BABIOLE SAUNIER, Maggy, F-68130 Jettingen (FR); BIZEC, Jean-Claude, F-68440 Habsheim (FR); WONG, Michelle, Pik-Han, Clifton, NJ 07013 (US); YEN, Shau-Fong, Atlanta, GA 30319 (US)
(74) Representative: de Weerd, Petrus G.W.
(86) International application number: PCT/EP2003/002156
(87) International publication number: WO 2003/074054

(56) References cited:
- EP-A- 0 406 791
- EP-A- 0 474 126
- EP-A- 0 737 478
- EP-A- 1 074 255
- WO-A-00/32234
- WO-A-00/66122
- WO-A-95/05176
- WO-A-96/13249
- US-A- 5 496 811

## Description

This invention relates to the use of topical ophthalmic compositions comprising an ascomycin and a carrier comprising a C₆ to C₁₂ fatty acid triglyceride in the manufacture of a medicament for the treatment of blepharitis.

Under "ascomycin" is to be understood ascomycin itself or a derivative, antagonist, agonist or analogue thereof, e.g. a compound of the FK 506 class.

Preferred ascomycins for use in the present invention include FK506 or a derivative, antagonist, agonist or analogue of FK506, which retain the basic structure and modulate at least one of the biological properties (for example immunological properties) of FK506 such as described in e.g. EP 184162, EP 315978, EP 323042, EP 423714, EP 427680, EP 465426, EP 474126, WO 91/13889, WO 91/19495, EP 484936, EP 532088, EP 532089, EP 569337, EP 626385, WO 93/5059 and the like; 33-epi-chloro-33-desoxy-ascomycin as disclosed in Example 66a in EP 427680 (hereinafter referred to as Compound A); {[1E-(1R,3R,4R)]1R,4S,5R,6S,9R,10E,13S,15S,16R,17S,19S,20S}-9-ethyl-6,16, 20-trihydroxy-4-[2-(4-hydroxy-3-methoxy-cyclohexyl)-1-methylvinyl]-15,17-dimethoxy-5,11,13,19-tetramethyl-3-oxa-22-aza-tricyclo[18.6.1.0(1,22)]heptacos-10-ene-2,8,21,27-tetraone as disclosed in Examples 6d and 71 in EP 569 337 (hereinafter referred to as Compound B); and {1R,5Z,9S,12S-[1E-(1R,3R,4R)],13R,14S,17R,18E, 21S,23S,24R,25S,27R}17-ethyl-1,14-dihydroxy-12-[2-(4-hydroxy-3-methoxy-cyclohexyl)-1-methylvinyl]-23,25-dimethoxy-13,19,21,27-tetramethyl-11,28-dioxa-4-aza-tricyclo[22.3.1.0(4,9)] octacosa-5,18-diene-2,3,10,16-tetraone, also known as 5,6-dehydro-ascomycin as disclosed in Example 8 in EP 626 385 (hereinafter referred to as Compound C); imidazolylmethyloxyascomycin, as disclosed in Example 1 and as compound of formula I in WO 97/08182 (hereinafter referred to as Compound D); 32-O-(1-hydroxyethylindol-5-yl)ascomycin, also known as Indolyl-ASC or L-732 531 as disclosed in Transplantation 65 (1998) 10-18, 18-26, on page 11, Figure 1 (hereinafter referred to as Compound E); or (32-deoxy-32-epi-N1-tetrazolyl)ascomycin, also known as ABT-281 as disclosed in J. Inv. Derm. 112 (1999), 729-738, on page 730, Figure 1 (hereinafter referred to as Compound F).

FK 506, Compounds A, B, C, D, E, and F are preferred ascomycins, more preferred are Compounds A, B, and C, especially Compound A. Particularly preferred is Compound A.

Ascomycins have a variety of useful pharmacological actions, e.g. treatment of blepharitis, and may be administered topically. However, inter alia because of their physicochemical properties, e.g. high molecular weight and lipophilicity the ascomycins have posed problems for topical administration. Furthermore due to the sensitivity of the delicate eye tissues, only ophthalmically acceptable components may be employed in ophthalmic compositions.

Formulations, e.g. in form of an ointment, for application to the skin comprising ascomycins have been described e.g. in EP 474126 or EP 1135163. However, due to the irritation potential of some of the components used, these ointments may not be topically applied to the eye.

Applicants have now found that ophthalmic compositions comprising an ascomycin and a carrier comprising a medium chain fatty acid triglyceride are highly efficient and well tolerated by the ocular tissue.

Preferred compositions of said king comprise 33-epi-chloro-33-desoxy-ascomycin.

The active agent may be in suspension, e.g. partially in suspension in the vehicle. Preferably the active agent is however dissolved, e.g. partially dissolved, in the vehicle.

If the active agent is suspended, it may preferably be used in a micronized form. The suspension may contain particles of ascomycin of from 5, e.g. from 10, to about 90, preferably to about 25 microns in diameter. The particles of the ascomycin may be produced in conventional manner, e.g. by grinding or milling. In case the active agent exists in different polymorphic or pseudo-polymorphic forms, the thermodynamic stable form is preferably used in a suspension type formulation.

The active agent is e.g. present in the compositions of this invention in an amount of from 0.01 to 5% by weight, e.g. 0.05 to 3% by weight, e.g. from 0.1 to 2% by weight, e.g. from about 0.2 to about 1% by weight based on the total weight of the composition.

The carrier comprises a medium chain triglyceride.

Medium chain fatty acid triglycerides are C₆ to C₁₂ fatty acid triglycerides, e.g. as known and commercially available under the trade name Acomed®, Myritol®, Captex®, Neobee®M5F, Miglyol®810, Miglyol®812, Mazol®, Sefsol®860, Sefsol®870. Especially preferred is the product Miglyol®812. Such medium chain fatty acid triglycerides are usually obtained from the oil extracted from the hard, dried fraction the endosperm of *Cocos nucifera* L. or from the dried endosperm of *Elaeis guineensis* Jaqu. They consist of a mixture of triglycerides of saturated fatty acids, mainly of caprylic acid and of capric acid, and contain not less than 95 percent of saturated fatty acids with 8 and 10 carbon atoms.

The medium chain fatty acid triglycerides may be present in an amount of 1 to 80% by weight based on the total weight of the composition, preferably about 10 to about 55% by weight.

Preferred compositions according to the invention include compositions which are in the form of an ointment and compositions which are in the form of an emulsion, in particular an oil-in-water emulsion.

The compositions according to the invention may therefore comprise a carrier which further comprises an ointment base. Suitable ointment bases include, for instance, ophthalmically acceptable oil and fat bases, such as
(a) natural wax e.g. white bees wax, carnauba wax, wool wax (wool fat), purified lanolin, anhydrous lanolin,
(b) petroleum wax e.g. solid paraffin, microcrystalline wax,
(c) hydrocarbons e.g. liquid paraffin, white petrolatum (e. g. white Protopet®), yellow petrolatum, or
(d) combinations thereof.

The above mentioned oil and fat bases are described, for instance, in the British Pharmacopoeia, Edition 2001, or in the European Pharmacopoeia, 3^{rd} Edition.

The ointment base may be present in an amount from 1 to about 95% by weight based on the total weight of the composition, e. g. 40 to 95% by weight. A preferred range for the percentage of ointment base is 45 to 90% by weight based on the total composition.

The ointment compositions according to the present invention may further comprise some water, preferably in an amount of less than 10% by weight based on the total amount of composition.

The ointment type compositions of the present invention may further comprise ophthalmically acceptable surfactants/emulsifiers.

The ophthalmic compositions of the instant invention include also compositions wherein the carrier further comprises water and an emulsifier instead of the ointment base.

Water may preferably be present in these compositions in amounts of 60 to 90% by weight, e.g. 70 to 85% by weight.

The emulsifier is preferably an ethoxylated C₁₆-C₁₈alkyl carboxylic acid (CAS Registry No. 68989-61-7). Corresponding emulsifiers are available under names like Pegoxol 7 stearate, Dion 37, Tefose 63, Tefose 70 or Stearox SP9 etc and exhibit in particular a good ocular tolerance. The emulsifier is used in amounts as required, e.g. 0.5 to 10% by weight, preferably 1 to 5% by weight.

The emulsifier may be accompanied by suitable co-emulsifiers, for example Lauroyl Macrogolglycerides, which are mixtures of mono-, di- and triesters of glycerol and lauric acid and mono- and diesters of macrogols (polyethylene glycols) having a mean molecular weight of e. g. between 300 and 1500. Suitable amounts range for example from 0.5 to 10% by weight, preferably 1 to 5% by weight.

The compositions of the present invention may further comprise an ophthalmically acceptable preservative. Suitable preservatives include
(a) a quaternary ammonium compound such as e.g. benzalkonium chloride (N-benzyl-N-(C₈₋C₁₈-alkyl)-N,N-dimethylammonium chloride), benzoxonium chloride, benzethonium chloride, cetrimide (hexadecyl-trimethylammonium bromide), sepazonium chloride, cetylpyridinium chloride, domiphen bromide (Bradosol®) or the like,
(b) alkyl-mercury salts of thiosalicylic acid, such as e.g. thiomersal, phenylmercuric nitrate, phenylmercuric acetate or phenylmercuric borate,
(c) parabens, such as e.g. methylparaben or propylparaben,
(d) alcohols, such as e.g. chlorobutanol, benzyl alcohol or phenyl ethyl alcohol,
(e) biguanide derivatives, such as e.g. chlorohexidine or polyhexamethylene biguanide,
(f) sodium perborate,
(g) imidazolidinyl urea as known and commercially available under the trade name Germal®II,
(h) sorbic acid,
(i) stabilized oxychloro complexes such as known and commercially available under the trade name Purite®,
(k) polyglycol-polyamine condensation resins, such as known and commercially available e.g. under the trade name Polyquart® from Henkel KGaA,
(I) stabilized hydrogen peroxide generated from a source of hydrogen peroxide for providing an effective trace amount of resultant hydrogen peroxide, e.g. sodium perborate tetrahydrate, and/or
(m) a mixture of any components (a) to (I).

Preferred preservatives are quaternary ammonium compounds, in particular benzalkonium chloride, cetrimide and phenyl ethyl alcohol. Where appropriate, a sufficient amount of preservative is added to the ophthalmic composition to ensure protection against secondary contaminations during use caused by bacteria and fungi, e.g. benzalkonium chloride and/or cetrimide are present in an amount of about 0.001-0.02%, or phenyl ethyl alcohol is present in an amount of about 0.05 to 1 %.

The compositions of the present invention, in particular the emulsion-type compositions, may also comprise a suitable amount, e. g. 1 to 10 % by weight, of a thickening agent, for example a suitable glycerol monostearate or a mixture of mainly glycerol monostearate together with variable amounts of di- and triacylglycerols like for instance Glycerol Monostearate 40-55, e.g. Geleol®, which is a mixture of 40 to 55% by weight of monoacylglycerols, 30 to 45% by weight of the diacylglycerols and 5 to 15% by weight of triacylglycerols obtained by partial glycerolysis of vegetable oils mainly comprising triacylglycerols of palmitic and/or stearic acid.

The compositions of the present invention may further comprise ophthalmically acceptable complexing agents such as
a) disodium-ethylenediamine tetraacetate, ethylenediamine tetraacetic acid (EDTA),
b) chelating agents having phosphonic acid or phosphonate groups, preferably organophosphonates, particularly amino tri(lower alkylene phosphonic acids) such as those known and commercially available from Monsanto Company, St. Louis, under the trade name Dequest®, or the like,
c) cyclodextrins, e.g. α-, β- or γ-cyclodextrin, e.g. alkylated, hydroxyalkylated, carboxy-alkylated or alkyloxycarbonyl-alkylated derivatives, or mono- or diglycosyl-α-, β- or γ-cyclodextrin, mono- or dimaltosyl-α-, β- or γ- cyclodextrin or panosyl-cyclodextrin, e.g. such as known and commercially available under the trade name Cavamax® or Cavasol® from Wacker Chemie, or
d) a mixture of components a) to c).

The compositions of the present invention may further comprise antioxidants such as ascorbic acid, acetylcysteine, cysteine, sodium hydrogen sulfite, butylated hydroxyanisole, butylated hydroxytoluene or alpha-tocopherol acetate.

The compositions of the present invention may further comprise ophthalmically acceptable stabilizers such thiourea, thiosorbitol, sodium dioctyl sulfosuccinate or monothioglycerol.

The compositions of the present invention may further comprise a buffer such as acetate, ascorbate, borate, hydrogen carbonate / carbonate, citrate, gluconate, lactate, phosphate, propionate and TRIS (tromethamine) buffers. Tromethamine and borate buffer are preferred buffers. The amount of a buffer added is, for example, that necessary to ensure and maintain a physiologically tolerable pH range. The pH range is typically in the range of from 5 to 9, preferably from 6 to 8.5 and more preferably from 6.5 to 8.2.

It will be appreciated that although the excipients have been described above by reference to a particular function any particular excipient may have alternative or multiple functions, e.g. cyclodextrin or a mixture of cyclodextrins may act as e.g. stabilizer, complexing agent and/or solubilizer.

Information on the properties, specifications and characteristics of the excipients are described e.g. in standard texts such as Fiedler, H.P.; 1996; Lexikon der Hilfsstoffe für Pharmazie, Kosmetik und angrenzende Gebiete; Editio Cantor Verlag Aulendorf (Germany), and Kibbe, A.H.; 2000; Handbook of Pharmaceutical Excipients, a joint publication of Pharmaceutical Press, London (UK), and American Pharmaceutical Association, Washington (US) as well as manufacturers' brochures.

Preferably, the compositions of the present invention are free of components such as perfumes or colorants.

Preferred compositions of the present invention consist essentially of an ascomycin, a medium chain triglyceride, an ointment base and a preservative.

The compositions of the present invention are stable, as indicated by conventional tests, e.g. under stressed conditions, such as a temperature cycling test at 5 to 30°C or 40°C if relevant, or several months, e.g. 1 to 12 months, at 30°C. A suitable temperature cycle test may be carried out for instance in the following way: the samples are kept 12 hours at 5°C and then 12 hours preferably at 30°C or 40°C if relevant (depending on the melting point of the tested ointments) for several month. The device type is for instance a Temperature Test Cabinet CTS T-40/25.

The ophthalmic compositions of the present invention may be prepared in conventional manner e.g. by mixing the preferably gamma-irradiated ascomycin powder with the appropriate excipients, e.g. by mixing the gamma-irradiated ascomycin powder with sterile filtered medium chain triglyceride, part of the ointment bases, e.g. the wool fat and/or liquid paraffin, and additional oil phase, if present, and ball mill the ascomycin in the liquid medium and subsequently aseptically add the ascomycin containing liquid medium to the matrix containing sterile preservative and remaining part of the ointment bases, e.g. white petrolatum.

It is also well possible, for example, to dissolve the ascomycin in preheated medium chain triglyceride, and to add the molten ointment base with the preservative. Thereafter a final sterile filtration through a 0.22 micron filter is performed,

Alternatively, the ascomycin may be dissolved e.g. in heated white petrolatum. The mixture may be filtered through a 0.2 micron filter, e.g. Durapore® membrane filter, and be allowed to cool and subsequently form a suspension. The suspension may be further ball milled to uniformly disperse the ascomycin.

The emulsion-type compositions may also be prepared in conventional manner, e.g. by dissolving the preferably gamma-irradiated ascomycin powder with the sterile filtered oily phase, in particular the medium chain triglyceride, adding the emulsifier and/or co-emulsifier and dispersing said mixture in an appropriate quantity of sterile water using conventional emulsification devices, e.g. a high-speed stirrer or an ultrasonic generator etc.

The present invention provides the use of a composition as defined above in the preparation of a medicament for the treatment of blepharitis e.g. chronic blepharitis, e.g. seborrhoeic blepharitis or allergic blepharitis, or staphylococcal blepharitis.

The utility of the compositions according to the invention can be observed in standard clinical tests such as the test set out below.

One animal test comprises a modified Draize test on three albino rabbits wherein the ocular tolerability after a single dose instillation of 50 microlitres of compositions of the present invention on eyelid or the ocular surface is shown for the 15 minutes after application then after 1, 2 and 7 days. The tolerability was based on visual examination considering the following parameters: discomfort as judged by blinking or partial/complete closure of the eye, duration of discomfort, discharge, redness of conjunctiva (palpebral and bulbar conjunctiva), chemosis of conjunctiva (swelling), degree of opacity of cornea and area of cornea involved, and pathological influence upon iris.

The compositions of the invention are found to be effective, well tolerated and allow a long-term treatment of patients, e.g. of those suffering from chronic blepharitis.

The exact amount of the ascomycin and of the composition to be administered depends on several factors, for example the desired duration of treatment and the rate of release of the ascomycin. Satisfactory results are obtained in larger mammals, e.g. humans, with the local application upon the eyelid to be treated or upon the ocular surfaces of the eye of a 0.01 to 5% by weight concentration of the ascomycin once or several times a day.

The following Examples illustrate the invention in more detail.

### Example 1 to 5 (ointments)

| Formulation | Ex. 1 | Ex. 2 | Ex. 3 | Ex. 4 | Ex. 5 |
|---|---|---|---|---|---|
| Compound A¹⁾ | 0.3-0.5 | 0.3-0.5 | 0.3-0.5 | 0.3-0.5 | 0.5 |
| Miglyol 812¹⁾ | 50 | 50 | 50 | 50 | 50 |
| Phenyl ethyl alcohol¹⁾ | 0.5 | 0.5 | - | - | - |
| Benzalkonium chloride¹⁾ | - | - | 0.010 | 0.015 | 0.010 |
| Wool fat¹⁾ | - | 5 | - | 5 | 5 |
| Liquid paraffin¹⁾ | - | - | - | - | - |
| White petrolatum¹⁾ | ad 100 | ad 100 | ad 100 | ad 100 | ad 100 |
| Preservative Efficacy Test with preservative Without preservative | USP³⁾ Failed | USP³⁾ Failed | EurP "A"⁴⁾ Failed | EurP "A"⁴⁾ Failed | EurP "A"⁴⁾ Failed |
| Ocular tolerance | moderate | moderate | moderate | moderate | moderate |

| | | | | | |
|---|---|---|---|---|---|
| ¹ amounts in weight percent ³ complies with the criteria for antimicrobial effectiveness according to the US Pharmacopeia ⁴ complies with the criteria "A" for antimicrobial effectiveness according to the European Pharmacopeia, chapter 5.1.3 | | | | | |

### Example 6 to 9 (ointments)

| Formulation | Ex. 6 | Ex. 7 | Ex. 8 Comparative | Ex. 9 |
|---|---|---|---|---|
| Compound A¹⁾ | 1.0 | 0.2 | 1.0 | 0.5-1.0 |
| Miglyol 812¹⁾ | 10 | 20 | - | 50 |
| Isopropyl myristate | - | - | 3.1 | - |
| Phenyl ethyl alcohol¹⁾ | 0.5 | - | 0.5 | 0.5 |
| Benzalkonium chloride¹⁾ | - | 0.015 | 0.010 | - |
| Wool fat¹⁾ | - | 6 | - | 10 |
| Microcrystalline wax | - | - | 12.9 | 13 |
| Liquid paraffin¹⁾ | - | 10 | 33.5 | 22 |
| White petrolatum¹⁾ | ad 100 | ad 100 | ad 100 | ad 100 |
| Preservative Efficacy Test | EurP "A"⁴⁾⁾ | EurP "A"⁴⁾ | - | - |
| with preservative Without preservative | Failed | Failed | - | - |
| Ocular tolerance | moderate | good | - | - |

| | | | | |
|---|---|---|---|---|
| ^{1), 3), 4)} cf. Examples 1 to 5 | | | | |

### Example 10 (emulsion-type composition)

| | |
|---|---|
| Composition | 0.3% Compound A |
| | 15.0% Medium chain triglycerides |
| | 2% Lauroyl Macrogologlycerides |
| | 3% Pegoxol 7 stearate (Tefose 63) |
| | 3% Glycerol monostearate 40-55 (Geleol) |
| | 1.25% Propylene glycol |
| | 0.5% Phenyl ethyl alcohol |
| | ad 100% Water |
| Appearence/ process | Solution |

| | |
|---|---|
| amounts in weight percent | |

Compound A is dissolved in the oily phase comprising the medium chain triglycerides. Then the water phase is added and the mixture is homogenized with an Ultra Turax homogenizer at 11000 rpm for about 30 seconds and finally stirred at 700 rpm for about 15 minutes.

The resulting composition exhibits a moderate ocular tolerance as proved in an animal model and meets the requirements as defined as European Pharmacopeia (Eur. Ph.) criteria B for ophthalmic preparations. The emulsion is stable at room temperature for at least 10 month (no phase separation occurs).

| | | |
|---|---|---|
| Composition / | 11 | 12 |
| Example | | |
| | | |
| Ingredients in wt. % | | |
| Compound A | 0.30 | 0.30 |
| Miglyol 812 | 50.00 | 50.00 |
| White petrolatum | 42.70 | 43.05 |
| Microcrystalline wax | 6.00 | 6.00 |
| Benzalkonium chloride | - | - |
| Chlorobutanol | - | - |
| Phenylethyl alcohol | 1.00 | 0.5 |
| Methylparaben | - | 0.1 |
| Propylparaben | - | 0.05 |
| Appearance | White ointment | White ointment |

## Claims

1. Use of an ophthalmic composition comprising an ascomycin and a carrier comprising a C₆ to C₁₂ fatty acid triglyceride in the manufacture of a medicament for the treatment of blepharitis.

2. Use according to claim 1 wherein said ascomycin is 33-epi-chloro-33-desoxy-ascomycin.

3. Use according to claim 1 wherein said carrier comprises an ointment base.

4. Use according to claim 1 wherein said carrier comprises water and an emulsifier.

5. Use according to claim 4, wherein said emulsifier is an ethoxylated C₁₆-C₁₈alkyl carboxylic acid.

6. Use according to claim 4 wherein said emulsifier is Pegoxol 7 stearate (Tefose 63).

7. Use according to claim 4 in form of an oil-in-water emulsion.

8. Use according to claim 1 in form of an ointment.

9. Use according to claim 1 further comprising a preservative.

10. Use of claim 9, wherein said preservative is selected from the group consisting of
(a) a quaternary ammonium compound such as e.g. benzalkonium chloride (N-benzyl-N-(C₈-C₁₈-alkyl)-N,N-dimethylammonium chloride), benzoxonium chloride, benzethonium chloride, cetrimide (hexadecyl-trimethylammonium bromide), sepazonium chloride, cetylpyridinium chloride, domiphen bromide (Bradosol®) or the like,
(b) alkyl-mercury salts of thiosalicylic acid, such as e.g. thiomersal, phenylmercuric nitrate, phenylmercuric acetate or phenylmercuric borate,
(c) parabens, such as e.g. methylparaben or propylparaben,
(d) alcohols, such as e.g. chlorobutanol, benzyl alcohol or phenyl ethyl alcohol,
(e) biguanide derivatives, such as e.g. chlorohexidine or polyhexamethylene biguanide,
(f) sodium perborate,
(g) imidazolidinyl urea as known and commercially available under the trade name Germal®II,
(h) sorbic acid,
(i) stabilized oxychloro complexes such as known and commercially available under the trade name Purite®,
(k) polyglycol-polyamine condensation resins, such as known and commercially available e.g. under the trade name Polyquart® from Henkel KGaA,
(l) stabilized hydrogen peroxide generated from a source of hydrogen peroxide for providing an effective trace amount of resultant hydrogen peroxide, e.g. sodium perborate tetrahydrate, and/or
(m) a mixture of any components (a) to (I).

11. Use of claim 10, wherein said preservative is selected from benzoxonium chloride, sodium perborate, phenyl ethyl alcohol, sorbic acid, Purite® and/or mixtures thereof.

12. Use of claim 1, wherein said blepharitis is seborrhoeic blepharitis or allergic blepharitis, or staphylococcal blepharitis.

## Patentansprüche

1. Verwendung einer ophthalmischen Zusammensetzung, die ein Ascomycin und einen Träger enthält, der ein C₆-C₁₂-Fettsäuretriglycerid umfasst, bei der Herstellung eines Arzneimittels für die Behandlung von Blepharitis.

2. Verwendung nach Anspruch 1, worin das Ascomycin 33-Epichlor-33-desoxyascomycin ist.

3. Verwendung nach Anspruch 1, worin der Träger eine Salbengrundlage umfasst.

4. Verwendung nach Anspruch 1, worin der Träger Wasser und einen Emulgator umfasst.

5. Verwendung nach Anspruch 4, worin der Emulgator eine ethoxylierte C₁₆-C₁₈-Alkylcarbonsäure ist.

6. Verwendung nach Anspruch 4, worin der Emulgator Pegoxol-7-stearat (Tefose 63) ist.

7. Verwendung nach Anspruch 4 in Form einer Öl-in-Wasser Emulsion.

8. Verwendung nach Anspruch 1 in Form einer Salbe.

9. Verwendung nach Anspruch 1, die ferner ein Konservierungsmittel umfasst.

10. Verwendung nach Anspruch 9, worin das Konservierungsmittel aus der Gruppe ausgewählt ist, die besteht aus
(a) einer quaternären Ammoniumverbindung, wie beispielsweise Benzalkoniumchlorid (N-Benzyl-N-(C₈-C₁₈-alkyl)-N,N-dimethylammoniumchorid), Benzoxoniumchlorid, Benzethoniumchlorid, Cetrimid (Hexadecyltrimethylammoniumbromid), Sepazoniumchlorid, Cetylpyridiniumchlorid, Domiphenbromid (Bradosol®) oder dergleichen,
(b) Alkylquecksilbersalzen von Thiosalicylsäure, wie beispielsweise Thiomersal, Phenylquecksilbernitrat, Phenylquecksilberacetat oder Phenylquecksilberborat,
(c) Parabenen, wie beispielsweise Methylparaben oder Propylparaben,
(d) Alkoholen, wie beispielsweise Chlorbutanol, Benzylalkohol oder Phenylethylalkohol,
(e) Biguanidderivaten, wie beispielsweise Chlorhexidin oder Polyhexamethylenbiguanid,
(f) Natriumperborat,
(g) Imidazolidinylharnstoff, wie er unter der Marke Germal® II bekannt und im Handel erhältlich ist,
(h) Sorbinsäure,
(i) stabilisierten Oxychlorkomplexen, wie sie unter der Marke Purite® bekannt und im Handel erhältlich sind,
(k) Polyglycol-Polyamin-Kondensationsharzen, wie sie beispielsweise unter der Marke Polyquart® von der Henkel KGaA bekannt und im Handel erhältlich sind,
(l) stabilisiertem Wasserstoffperoxid, wie es aus einer Quelle für Wasserstoffperoxid gebildet wird, um für eine wirksame Spurenmenge an erhaltenden Wasserstoffperoxid zu sorgen, beispielsweise Natriumperborattetrahydrat, und/oder
(m) einem Gemisch aus irgendeiner der Komponenten (a) bis (I).

11. Verwendung nach Anspruch 10, worin das Konservierungsmittel ausgewählt ist aus Benzoxoniumchlorid, Natriumperborat, Phenylethylalkohol, Sorbinsäure, Purite® und/oder Gemischen hiervon.

12. Verwendung nach Anspruch 1, worin die Blepharitis eine seborrhöische Blepharitis oder eine allergische Blepharitis oder eine staphylococcale Blepharitis ist.

## Revendications

1. Utilisation d'une composition ophtalmique constituée d'une ascomycine et d'un support comprenant un triglycéride d'acide gras en C₆ à C₁₂ dans la préparation d'un médicament pour le traitement de la blépharite.

2. Utilisation selon la revendication 1, dans laquelle ladite ascomycine est la 33-épi-chloro-33-désoxy-ascomycine.

3. Utilisation selon la revendication 1, dans laquelle ledit support comprend une base de pommade.

4. Utilisation selon la revendication 1, dans laquelle ledit support comprend de l'eau et un émulsionnant.

5. Utilisation selon la revendication 4, dans laquelle ledit émulsionnant est un acide alkylcarboxylique éthoxylé en C₁₆ à C₁₈.

6. Utilisation selon la revendication 4, dans laquelle ledit émulsionnant est le stéarate de Pegoxol 7 (Tefose 63).

7. Utilisation selon la revendication 4, sous forme d'une émulsion huile-dans-eau.

8. Utilisation selon la revendication 1 sous forme d'une pommade.

9. Utilisation selon la revendication 1, comprenant en outre un conservateur.

10. Utilisation selon la revendication 9, dans laquelle ledit conservateur est choisi dans le groupe constitué par :
(a) un composé d'ammonium quaternaire tel que, par exemple, le chlorure de benzalkonium (chlorure de N-benzyl-N-(alkyle en C₈ à C₁₈)-N,N-diméthylammonium), le chlorure de benzoxonium, le chlorure de benzéthonium, le cétrimide (bromure d'hexadécyl-triméthylammonium), le chlorure de sépazonium, le chlorure de cétylpyridinium, le bromure de domiphène (Bradosol®), etc.,
(b) des sels alkylmercuriques d'acide thiosalicylique, tels que, par exemple, le thiomersal, le nitrate phénylmercurique, l'acétate phénylmercurique ou le borate phénylmercurique,
(c) des parabènes, tels que, par exemple, le méthylparabène ou le propylparabène,
(d) des alcools, tels que, par exemple, le chlorobutanol, l'alcool benzylique ou l'alcool phényléthylique,
(e) des dérivés de biguanide, tels que, par exemple, la chlorohexidine ou le biguanide de polyhexaméthylène,
(f) le perborate de sodium,
(g) l'imidazolidinylurée, telle que connue et disponible dans le commerce sous le nom commercial de Germal® II,
(h) l'acide sorbique,
(i) des complexes oxychloro stabilisés, tels que connus et disponibles dans le commerce sous le nom commercial de Purite®,
(k) des résines de condensation de polyglycol-polyamine, telles que connues et disponibles dans le commerce, par exemple, sous le nom commercial de Polyquart® auprès de Henkel KGaA,
(1) un peroxyde d'hydrogène stabilisé généré à partir d'une source de peroxyde d'hydrogène, pour fournir une quantité efficace à l'état de traces de peroxyde d'hydrogène résultant, par exemple le perborate de sodium tétrahydraté, et/ou
(m) un mélange de n'importe lesquels des composants (a) à (1).

11. Utilisation selon la revendication 10, dans laquelle ledit conservateur est choisi parmi le chlorure de benzoxonium, le perborate de sodium, l'alcool phényléthylique, l'acide sorbique, la Purite® et/ou leurs mélanges.

12. Utilisation selon la revendication 1, dans laquelle ladite blépharite est une blépharite séborrhéique ou une blépharite allergique, ou une blépharite staphylococcique.
